Europäisches Patentamt

European Patent Office

Office européen des brevets

(19)

(11) Veröffentlichungsnummer: **0 077 923**
**A1**

(12) **EUROPÄISCHE PATENTANMELDUNG**

(21) Anmeldenummer: **82108741.8**

(22) Anmeldetag: **21.09.82**

(51) Int. Cl.³: **A 61 B 10/00**
**A 61 B 1/00, A 61 B 1/30**
**G 10 K 11/34**

(30) Priorität: **15.10.81 DE 3141022**

(43) Veröffentlichungstag der Anmeldung:
**04.05.83 Patentblatt 83/18**

(84) Benannte Vertragsstaaten:
**DE FR GB**

(71) Anmelder: **SIEMENS AKTIENGESELLSCHAFT**
**Berlin und München Wittelsbacherplatz 2**
**D-8000 München 2(DE)**

(72) Erfinder: **Hetz, Walter**
**Adam-Kraft-Strasse 17**
**D-8520 Erlangen(DE)**

(54) In einen Körper einführbare Ultraschall-Sonde.

(57) In einem Körper einführbare Ultraschall-Sonde mit einem Sondenrohr (5), an dem ein Optikeinsatz (10) und ein Ultraschall-Array (7) in zwei radial und auch in Rohrlängsrichtung gegeneinander versetzten Schichten angeordnet sind. Die in Richtung distales Rohrende (6) vor dem Ultraschall-Array mündende Stirnfläche (9) des Optikeinsatzes ragt nur so weit über die Applikationsfläche (Ultraschallelemente 21) des Ultraschall-Arrays, dass der Raum über dem Ultraschall-Array hinreichend ausleuchtbar ist. Bei vollständiger Ausfüllung des bezüglich Ultraschall-Array und Optikeinsatz proximal gelegenen Rohrraumes mit einem Bündel (11) der Signalleitungen (25,28) des Ultraschall-Arrays soll der Gesamtdurchmesser des Sondenrohres mit ein- oder aufgesetztem Optikeinsatz (10) so gering werden, dass damit insbesondere Blasen-Endoskopie bei männlichen Patienten durchgeführt werden kann.

./...

Croydon Printing Company Ltd

FIG 1

FIG 2

SIEMENS AKTIENGESELLSCHAFT          Unser Zeichen
Berlin und München                  VPA 81 P 5096  E

## In einen Körper einführbare Ultraschall-Sonde

Die Erfindung bezieht sich auf eine in einen Körper, z.B. dem eines Patienten, einführbare Ultraschall-Sonde gemäß den Merkmalen des Oberbegriffes des Patentanspruches 1.

Eine Ultraschall-Sonde dieser Art ist durch die DE-PS 23 05 501 vorbekannt. Es besteht der Wunsch, derartige Ultraschall-Sonden im Zusammenhang mit der Endoskopie einzusetzen. In einem solchen Falle muß also die Ultraschall-Sonde wenigstens zusammen mit einem endoskopischen Optikeinsatz in die Körperhöhle des Patienten eingeführt werden. Der endoskopische Optikeinsatz ermöglicht es dem Arzt, Einblick von außen zum Untersuchungsort im Innern des Patientenkörpers zu nehmen, an dem die Ultraschall-Sonde plaziert werden soll. Ist ein geeigneter Untersuchungsort gefunden, so wird der Ultraschall-Applikator zwecks Aufzeichnung von Querschnittsbildern in Betrieb genommen. Die Art der Abtastung ist beliebig; es kann sich also um reinen Linear-Scan oder auch um elektronisch erzeugten Sektor-Scan handeln. Ebensogut ist ein C-Scan od.dgl. möglich. Eine Ultraschall-Sonde, die zusammen mit einem Optikeinsatz bei Endoskopen eingesetzt wird, ist an sich aus der DE-OS 30 09 482 bereits vorbekannt. Bei dem Ultraschall-Wandler dieser Ultraschall-Sonde handelt es sich jedoch um einen mechanisch geschwenkten Ultraschallkopf für Sektorabtastung. Ein solcher Abtastkopf benötigt allenfalls eine Signalleitung für die Erregung und für den Echoempfang und eine Leitung für einen Winkelgeber. Abgesehen von dem Antriebsdraht oder der Antriebswelle

Kue 5 Kof / 14.10.1981

für die Durchführung der Schwenkbewegung des Schallkopfes sowie dem Winkelgeber wird kein Raum für weiteres Zubehör des Ultraschall-Wandlers benötigt. Aus
diesem Grunde kann der Ultraschall-Wandler zusammen
mit einem Optikeinsatz vollständig im Sondenrohr untergebracht werden, das auch den Wandler trägt.

Bei Einsatz eines Ultraschall-Arrays ist eine solche
Bauweise nicht so ohne weiteres möglich. Ein Ultraschall-Array erlaubt eine großflächigere Abtastung des
Untersuchungsobjektes, insbesondere auch solcher Stellen, die nahe am Array liegen. Beim Sektor-Abtastkopf
ergeben sich brauchbare Bilder erst in einem gewissen
Abstand von der Spitze des Sektorzeilenfeldes, d.h. vom
Schallkopf.

Um den Vorteil eines Ultraschall-Arrays voll ausnutzen
zu können, sollte das Array wenigstens eine solche Zahl
von Wandlerelementen aufweisen, daß sich ein brauchbar
aufgelöstes Ultraschallbild mit hinreichender Breite er
gibt. Die Praxis zeigt,daß brauchbare Bilder dann geliefert werden, wenn die Zahl der Wandlerelemente bei
wenigstens etwa 40 bis 50 Einzelelementen liegt. Diese
schon relativ hohe Zahl von Einzelelementen, die in
sich wieder feingeteilt sein können (z.B. nach Art der
DE-PS 28 29 570 in zwei oder vorzugsweise noch mehr,
wie z.B. acht, Teilschwinger), bedingt jedoch eine entsprechend hohe Zahl von Signalleitungen. Handelsübliche
Signalleitungen sind im allgemeinen recht dick (sie haben einen Durchmesser im Bereich $\emptyset = 0,6$ mm), da sie
zur gegenseitigen HF-Entstörung neben der normalen Isolierschicht aus z.B. Teflon auch noch mit einem HF-
Schutzmantel aus z.B. vergoldetem Kupferdraht versehen
sind.

Aufgabe vorliegender Erfindung ist es, ein Sondenrohr
für ein Ultraschall-Array mit einer Vielzahl von Signalleitungen und einem Optikeinsatz aufzubauen, das trotz
des zusätzlichen Optikeinsatzes und trotz der Tatsache,
daß eine Vielzahl von Leitungen geführt werden muß, im
Außendurchmesser klein ist. Die Ultraschall-Sonde soll
dabei insbesondere im Zusammenhang mit der Endoskopie
in übliche Endoskopführungsrohre einsetzbar sein. Die
Abmessungen sollen auch so sein, daß ein Einsatz in
solchen Endoskopführungsrohren möglich ist, die zu
Blasenuntersuchungen in der Urologie verwendet werden.
Das Sondenrohr und der Optikeinsatz sollen dann gemeinsam in ein solches Endoskopführungsrohr passen, das mit
den üblichen maximal zugelassenen Abmessungen selbst
durch die Harnröhre des Penis eines männlichen Patienten gefahrlos in die Harnblase einführbar ist.

Die Aufgabe wird erfindungsgemäß mit den kennzeichnenden
Merkmalen des Patentanspruches 1 gelöst.

Die Erfindung ermöglicht einen besonders kompakten Aufbau eines Sondenrohres zusammen mit einem Optikeinsatz.
Dem Wunsch nach kleinen Abmessungen wird damit in optimaler Weise Rechnung getragen; die vorstehend aufgezeigte Aufgabenstellung wird optimal gelöst.

Weitere Vorteile und Einzelheiten der Erfindung ergeben
sich aus der nachfolgenden Beschreibung eines Ausführungsbeispieles anhand der Zeichnung in Verbindung mit
den Unteransprüchen.

Es zeigen:
Figur 1 ein handelsübliches Endoskop für z.B.
urologische Untersuchungen, mit passendem Optikeinsatz und einem dazu passend
gestalteten Sondenrohr mit einem Ultraschall-Array,

Figur 2 einen vergrößerten Ausschnitt des di-
          stalen Endes des im Endoskopführungs-
          rohr geführten Sondenrohres,

Figur 3 einen Querschnitt durch Endoskopführungsrohr, Lichtleitereinsatz und Son-
          denrohr an der Stelle III-III,

Figur 4 einen ebensolchen Querschnitt an der
          Stelle IV-IV.

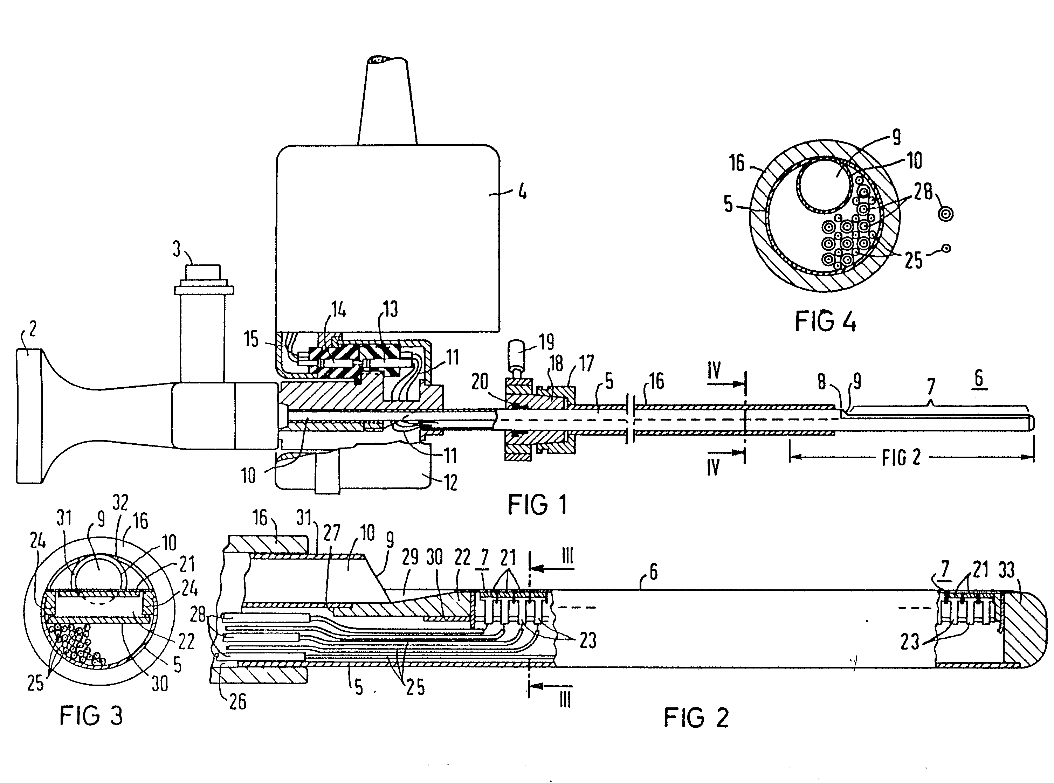

Die Figur 1 zeigt ein Endoskop 1, das proximal mit einem Okular 2, einem Kaltlichtanschluß 3 und einem Elektronikteil 4 für die elektrische Signalbedienung eines Ultraschall-Arrays sowie distal mit einem Sondenrohr 5 bestückt ist. Das Sondenrohr 5, das aus ca. 0,2
bis 0,3 mm dickem rostfreiem Stahl besteht, ist am
äußersten distalen Ende 6 auf der oberen Seite abgeflacht (in dem z.B. dieses Rohrendstück abgestuft ausgebildet ist). Auf dem abgeflachten Teil des Rohrendstückes 6 sitzt das Ultraschall-Array 7. An der Stelle 8,
an der das Sondenrohr 5 in den Flachteil übergeht, mündet das z.B. abgeschrägte distale Ende 9 eines Optikeinsatzes 10, so wie er üblicherweise in der Endoskopie
verwendet wird. Der Optikeinsatz 10, der aus einer im
einzelnen nicht dargestellten Optik mit Lichtleitfasern
für Kaltlicht besteht, ist am proximalen Ende einerseits
mit dem Okular 2 und andererseits mit dem Anschluß 3
für Kaltlicht verbunden. Er verläuft im oberen Raum des
Sondenrohres 5 parallel zur Rohrachse. Der untere Raum
des Sondenrohres ist, wie anhand der Figuren 2 bis 4
noch näher erläutert wird, in besonderer Weise mit den
Signalleitungen des Ultraschall-Arrays 7 ausgefüllt. Die
se Signalleitungen, die in der Figur 1 nur als proximal
auslaufendes Bündel 11 im Verbundstück 12 für das Elektronikteil 4 angedeutet sind, sind am proximalen Ende über entsprechend ausgebildete Kontaktstecker 13 mit Gegensteckkontakten 14 der Anschlußleitungen 15 des Elektronikteiles 4 verbunden. Das Verbundstück 12 besitzt ein metallisches Kapselgehäuse. Entsprechend ist auch der Elektronikteil 4 metallisch (z.B. Kunststoffgehäuse mit Innenmetallisierung) abgekapselt. Die elektrischen Signalleitungen sind daher nach außen hin elektrisch gut isoliert und auch gegen störenden Hochfrequenzeinfall von außen gut abgesichert.

Das Endoskop der Figur 1 umfaßt in üblicher Weise auch noch ein Endoskopführungsrohr 16. Dieses Führungsrohr 16 wird normalerweise zuerst in die Körperhöhle des Patienten eingeführt. Erst anschließend wird durch das Führungsrohr jener Teil des Endoskopes eingeführt, mit dem das Körperinnere untersucht werden soll. Im Falle der Figur 1 wird also das Führungsrohr 16 z.B. durch die Harnröhre des Penis eines männlichen Patienten bis zum Eingang der Harnblase eingeführt. Dann wird durch das Führungsrohr 16 das Sondenrohr 5 mit Ultraschall-Array 6 und Optikeinsatz 10 in die Blase eingeführt. Das Sondenrohr 5 läßt sich mit dem Endoskopführungsrohr 16 kuppeln. Zu diesem Zweck weist das Endoskopführungsrohr 16 am proximalen Ende einen Kupplungsschaft 17 auf, der zu einem Kupplungsteil 18 mit Verriegelungshebel 19 am Sondenrohr 5 paßt. Mit 20 ist ein Dichtungsring des Kupplungsstückes angedeutet.

In der vergrößerten Darstellung der Figur 2 trägt das distale Sondenrohrende 6 ein Ultraschall-Array 7 mit z.B. achtundvierzig Ultraschallelementen 21 aus piezoelektrischem Material, die, wie üblich, auf einem Dämpfungskörper 22, z.B. aus einem Kunstharz mit Füllstoffen, aufgebracht sind. Jedes der Wandlerelemente 21 ist nach Art der DE-PS 28 29 570 in z.B. vier Teilschwinger unterteilt, die über gemeinsame Kontaktfahnen 23 elektrisch miteinander verbunden sind. Am
unteren Ende der Kontaktfahnen 23 sind schließlich die
distalen Enden der Signalleitungen des proximalen Bündels 11 für die einzelnen Ultraschallwandlerelemente
angelötet. Die Signalleitungen sind an dieser Stelle
lediglich isoliert; sie weisen also keine zusätzliche
Hochfrequenz (HF)-Abschirmung auf. Signalleitungen oder
Signalleitungsteile ohne zusätzliche HF-Isolierung sind
in den Figuren 2 bis 4 mit 25 bezeichnet. Mit dem somit
geringeren Durchmesser (ca. 0,25 mm) dieser Signalleitungen oder Signalleitungsteile 25 gelingt es, den entlang dem abgeflachten distalen Ende des Sondenrohres
unterhalb dem Ultraschall-Array verbleibenden Hohlraum
optimal mit Leitungen auszufüllen. Eine HF-Abschirmung
der Leitungen erfolgt erst im Raum 26 hinter der Stoßstelle 27 zwischen Ultraschall-Array und dem distalen
Ende des Optikeinsatzes 10. Ab dieser Stoßstelle ist
gerade wieder so viel Raum vorhanden, daß in diesem
Raum entlang dem restlichen Teil des Rohres bis zum
Verbundstück 12 für das Elektronikteil 4 abgeschirmte
Signalleitungen oder Signalleitungsteile, die hier speziell mit 28 gekennzeichnet sind, und nicht abgeschirmte Signalleitungen oder Signalleitungsteile, die wieder die Kennziffer 25 tragen, in vermischter Form weitergeleitet werden können. Dieser Sachverhalt ist im
Querschnitt der Figur 4 dargestellt, wo nicht abgeschirmte Leitungen oder Leitungsteile 25 so vollständig zwischen abgeschirmten Leitungen oder Leitungsteilen 28 zu liegen kommen, daß sie ihrerseits durch abgeschirmte Leitungen oder Leitungsteile 28 abgeschirmt
sind.

In der Ausschnittsvergrößerung der Figur 2 ist der
Dämpfungskörper 22 des Ultraschall-Arrays 7 proximal
an der Stoßfläche zur Stirnfläche 9 des Optikeinsatzes so abgeschrägt, daß sich dort eine schräge flache Mulde 29 für den Lichtaustritt des Optikeinsatzes ergibt. Im Querschnittsbild der Figur 3 sind die Bauteile 24 Seitenstücke aus Araldit. Das Bauteil 30 ist eine Leiterplatte.

Aus dem Querschnittsbild der Figur 3 ist auch noch folgendes zu entnehmen:

Der Optikeinsatz 10 sitzt in einem Rohr 31. Das Sondenrohr 5 hat bis zum Übergang zum abgeflachten distalen Endteil 6, dh. bis zur Stirnfläche 9 des Optikeinsatzes 10, die Form eines Vollrohres. Dieses Vollrohr weist jedoch entlang seiner oberen Fläche parallel zur Rohrachse einen schmalen Schlitz 32 auf. Dieser Schlitz 32, der in der Figur 3 angedeutet ist, ist so gewählt, daß das eingesetzte Rohr 31 für den Optikeinsatz 10 durch den Schlitz hindurch nach oben bündig mit dem Umfang des Vollrohres abschließt. Auf diese Weise wird vermieden, daß sich an der Stoßstelle zwischen Sondenrohr 5 und Rohr 31 für den Optikeinsatz die Wandstärken beider Rohre addieren. Das Rohr 31 für den Optikeinsatz kann also geringfügig nach oben versetzt werden, wodurch wieder mehr Platz für die Leitungsführung im Raum 26 des Sondenrohres 5 verbleibt. Eine solche Spezialausbildung fördert also die optimale Abstimmung zwischen Inneneinrichtung des Sondenrohres und Außendurchmesser dieses Sondenrohres. Werden hingegen Rohre 31 für den Optikeinsatz mit sehr dünner Wandstärke verwendet, so kann auf die Anbringung eines Öffnungsschlitzes 32 im Sondenrohr verzichtet werden. In diesem Falle liegt das Rohr 31 für den Optikeinsatz lediglich an der Innenwandung des äußeren Sondenrohres 5 an. Die Wandstärken beider Rohre addieren sich in diesem Falle. Eine solche Ausführungsform soll selbstverständlich mit unter die Erfindung fallen. In der Figur 2 ist schließlich das äußerste distale Ende des abgeflachten Rohrteiles 6 mit einem Gießharzverschluß 33 versehen.

Im Ausführungsbeispiel der Figuren 1 bis 4 läßt sich ein Optikeinsatz 10, der in einem Hüllrohr 31 mit einem äußeren Rohrdurchmesser von ca. 2,6 mm eingelagert ist, mit einem Paket aus HF-abgeschirmten bzw. zwischengelagerten, nicht abgeschirmten Verbindungsleitungen eines Ultraschall-Arrays mit z.B. 48 Wandlerelementen der vorstehend beschriebenen Art so zusammensetzen, daß das Gesamtpaket in ein Sondenrohrvollteil 5 mit einem optimal niedrigen Außendurchmesser von ca. 5,4 mm paßt. Ein Sondenrohr mit einem solchen Aussendurchmesser paßt wiederum auf jeden Fall in handelsübliche Führungsrohre für Blasenendoskopie von männlichen Patienten. Der Außendurchmesser dieser Führungsrohre beträgt bei 0,8 mm Wandstärke 21 Charrier, was 7 mm entspricht. Das Ultraschall-Array selbst ist z.B. als 7 MHz-Array ausgebildet.

Patentansprüche

1. In einen Körper, z.B. dem eines Patienten, einführbare Ultraschall-Sonde, bestehend aus einem Sondenrohr, an dem ein Ultraschall-Array angeordnet ist, das mit seiner Längsachse in Richtung des Sondenrohres ausgerichtet ist und in dem elektrische Verbindungsleitungen für den Anschluß des Ultraschall-Arrays an eine körperexterne Ultraschall-Schnittbild-Anzeigevorrichtung verlaufen, d a d u r'c h   g e k e n n z e i c h n e t , daß das am Sondenrohr (5) angeordnete Ultraschall-Array (7), wie es an sich schon für anders gestaltete Ultraschall-Wandler bekannt ist, in Kombination mit einem endoskopischen Optikeinsatz (10) den Bestandteil eines Endoskopes (1) bildet in der Weise, daß Optikeinsatz (10) und Ultraschall-Array (7) in zwei radial und auch in Rohrlängsrichtung gegeneinander versetzten Schichten angeordnet sind derart, daß die in .Richtung distales Rohrende (6) vor dem Ultraschall-Array mündende Stirnfläche (9) des Optikeinsatzes nur so weit über die Applikationsfläche (Ultraschallelemente 21) des Ultraschall-Arrays ragt, daß einerseits der Raum über dem Ultraschall-Array hinreichend ausleuchtbar ist und andererseits bei weitgehend vollständiger Ausfüllung des Rohrraumes unterhalb Ultraschall-Array und Optikeinsatz mit dem Bündel (11) der in Richtung proximalem Rohrende laufenden elektrischen Verbindungsleitungen (25, 28) des Ultraschall-Arrays der Gesamtdurchmesser des Sondenrohres mit ein- oder aufgesetztem Optikeinsatz (10) für Endoskopiezwecke optimal gering wird.

2. Ultraschall-Sonde nach Anspruch 1, d a d u r c h   g e k e n n z e i c h n e t ,   daß ein Vollrohr als Sondenrohr (5) am distalen Rohrende (6) stufenförmig abgeflacht ist und daß der Optikeinsatz (10) im oberen Raum des Vollrohres so einsetzbar ist, daß er mit seiner Stirnfläche (9) an der Übergangsstelle (8) zwischen Vollrohr und abgeflachtem distalen Rohrende (6) mündet derart, daß die Stirnfläche (9) das auf dem abgeflachten distalen Rohrteil (6) angeordnete Ultraschall-Array (7) in der zur hinreichenden Ausleuchtung erwünschten Weise überragt.

3. Ultraschall-Sonde nach Anspruch 2, d a d u r c h g e k e n n z e i c h n e t , daß der Optikeinsatz (10) selbst in einem Rohr (31) angeordnet ist, welches Rohr an der Innenwand des Vollrohres des Sondenrohres (5) anliegt, so daß sich die Wandstärken beider Rohre addieren.

4. Ultraschall-Sonde nach Anspruch 2, d a d u r c h g e k e n n z e i c h n e t , daß der Optikeinsatz (10) selbst in einem Rohr (31) angeordnet ist, welches Rohr (31) nach Einsetzung in das Sondenrohr (5) in einen Schlitz (32), der in Längsrichtung des Vollrohres verläuft, eingreift, derart, daß das Rohr (31) für den Optikeinsatz (10) mit seiner Außenwandung bündig mit dem Umfang des Vollrohres (5) abschließt.

5. Ultraschall-Sonde nach einem der Ansprüche 1 bis 4, d a d u r c h g e k e n n z e i c h n e t , daß der Optikeinsatz (10) an der Stoßstelle (8) zum Ultraschall-Array (7) eine schräg zur Normale mündende Stirnfläche (9) aufweist.

6. Ultraschall-Sonde nach einem der Ansprüche 1 bis 5, d a d u r c h g e k e n n z e i c h n e t , daß das Ultraschall-Array einen Dämpfungskörper (22) für die Ultraschallelemente (21) umfaßt, der proximal an der Stoßfläche zur Stirnfläche (9) des Optikeinsatzes (10) so abgeschrägt ist, daß sich dort eine schräge flache Mulde (29) für den Lichtaustritt des Optikeinsatzes ergibt.

7. Ultraschall-Sonde nach einem der Ansprüche 1 bis 6, d a d u r c h   g e k e n n z e i c h n e t ,   daß im abgeflachten distalen Endteil (6) des Sondenrohres (5) im Hohlraum unterhalb des Ultraschall-Arrays (7) im wesentlichen nur solche Signalleitungen oder Signalleitungsabschnitte (25) verlaufen, die an dieser Stelle keine oder noch keine Hochfrequenzabschirmung aufweisen und die deshalb geringere Durchmesser aufweisen als die hochfrequenzmäßig abgeschirmten Leitungen oder Leitungsteile.

8. Ultraschall-Sonde nach einem der Ansprüche 1 bis 7, d a d u r c h   g e k e n n z e i c h n e t ,   daß im Hohlraum (26) des Sondenrohres (5) unterhalb des Optikeinsatzes (10) Verbindungsleitungen des Ultraschall-Arrays (7) verlaufen, die im wesentlichen hochfrequenzmäßig abgeschirmt sind.

9. Ultraschall-Sonde nach Anspruch 8, d a d u r c h   g e k e n n z e i c h n e t ,   daß in diesem Raum (26) hochfrequenzmäßig abgeschirmte Signalleitungen oder Signalleitungsabschnitte (28) zusammen mit nicht abgeschirmten Signalleitungen oder Signalleitungsabschnitten (25) weitergeleitet werden, wobei nicht abgeschirmte Leitungen oder Leitungsabschnitte (25) so vollständig zwischen abgeschirmten Leitungen oder Leitungsabschnitten (28) liegen, daß sie ihrerseits durch abgeschirmte Leitungen oder Leitungsabschnitte (28) gegeneinander abgeschirmt sind.

Europäisches Patentamt

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A,D | --- <br> DE-A-2 305 501 (SIEMENS AG) <br> * Seite 3, Zeilen 13-24; Seite 5, Zeile 18 - Seite 6, Zeile 4; Seite 7, Zeilen 10-16; Abbildung 3 * | 1 | A 61 B 10/00 <br> A 61 B 1/00 <br> A 61 B 1/30 <br> G 10 K 11/34 |
| A,D | --- <br> DE-A-3 009 482 (OLYMPUS OPTICAL CO., LTD.) <br> * Seite 24, Zeilen 6-24; Abbildung 6 * | 1 | |
| A | --- <br> IEEE TRANSACTIONS ON SONICS AND ULTRASONICS, Band SU-27, Nr. 6, November 1980, Seiten 277-286, New York, USA <br> R.W. MARTIN et al.: "An ultrasonic catheter for intravascular measurement of blood flow: technical details" * Seiten 279-281, Abschnitt "General"; Abbildungen 3-5 * | 8 | |
| A,P | --- <br> EP-A-0 039 851 (OLYMPUS OPTICAL CO., LTD.) <br> * Zusammenfassung; Seite 3, Zeile 7 - Seite 4, Zeile 3; Abbildungen 1-6 * | 1,2 | RECHERCHIERTE SACHGEBIETE (Int. Cl. ³) <br><br> A 61 B 10/00 <br> G 10 K 11/34 <br> A 61 B 1/00 |
| A | --- <br> DE-A-2 950 203 (OLYMPUS OPTICAL CO., LTD.) <br> * Seite 8, Zeile 10 - Seite 9, Zeile 15; Abbildung 2 * | 1 | |
| | ---      -/- | | |

Der vorliegende Recherchenbericht wurde für alle Patentansprüche erstellt.

| Recherchenort <br> DEN HAAG | Abschlußdatum der Recherche <br> 21-01-1983 | Prüfer <br> RIEB D.K. |
|---|---|---|

Europäisches Patentamt

**EUROPÄISCHER RECHERCHENBERICHT**

**0077923**
Nummer der Anmeldung

EP 82 10 8741

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| P,A | US-A-4 327 738 (P.S. GREEN et al.) * Zusammenfassung; Spalte 3, Zeilen 19-61; Spalte 5, Zeilen 3-21; Spalte 6, Zeilen 16-27; Abbildungen 1-7 * | 1,2,6, 8 | |
| E | EP-A-0 061 332 (OLYMPUS OPTICAL CO., LTD.) * Seite 4, Zeilen 7-21; Seite 8, Zeile 14 - Seite 9, Zeile 3; Seite 12, Zeile 13 - Seite 13, Zeile 5; Abbildungen 4,7,9 * | 1,3,5 | |

RECHERCHIERTE SACHGEBIETE (Int. Cl. 3)

Der vorliegende Recherchenbericht wurde für alle Patentansprüche erstellt.

| Recherchenort DEN HAAG | Abschlußdatum der Recherche 21-01-1983 | Prüfer RIEB D.K. |
|---|---|---|